# FASCICULE DE BREVET EUROPEEN

(11) **EP 2 699 248 B1**
(45) Date de publication et mention de la délivrance du brevet: **08.02.2017**
(21) Numéro de dépôt: 12714726.2
(22) Date de dépôt: 19.04.2012
(51) Int. Cl.: A61K 35/16, A61M 1/02

(54) **PROCÉDÉ DE PRÉPARATION D'UN PRODUIT PLASMATIQUE DÉPLÉTÉ EN UN OU PLUSIEURS FACTEURS THROMBOGENES**
VERFAHREN ZUR HERSTELLUNG EINES VERARMTEN PLASMAMATERIALS MIT EINEM ODER MEHREREN THROMBOGENEN FAKTOREN
METHOD FOR PREPARING A DEPLETED PLASMA MATERIAL CONSISTING OF ONE OR MORE THROMBOGENIC FACTORS

(30) Priorité: 20.04.2011 FR 1153438
(43) Date de publication de la demande: 26.02.2014
(73) Titulaire: Laboratoire Français du Fractionnement et des Biotechnologies, 91940 Les Ulis (FR)
(72) Inventeur: OLLIVIER, Monique, F-94270 Le Kremlin Bicetre (FR); PAOLANTONACCI, Philippe, F-91190 Gif Sur Yvette (FR)
(74) Mandataire: Pontet Allano & Associes
(86) Numéro de dépôt international: PCT/EP2012/057228
(87) Numéro de publication internationale: WO 2012/143484

(56) Documents cités:
- EP-A1- 0 512 883
- WO-A1-02/092632
- WO-A2-2007/077365
- US-A- 4 272 521
- US-A- 5 099 003
- US-A- 5 164 487

## Description

### Domaine technique

La présente invention concerne un procédé de préparation d'un produit plasmatique déplété en un ou plusieurs facteurs thrombogènes. La présente invention concerne également de tels produits plasmatiques obtenus par ledit procédé.

### Etat de la technique

Le plasma humain comprend une grande quantité de composés susceptibles de présenter un intérêt thérapeutique et/ou prophylactique tels que, par exemple, les facteurs de la coagulation, les immunoglobulines ou l'albumine. C'est pourquoi de nombreux procédés de purification de produits plasmatiques ont été développés, en mettant en oeuvre des techniques extrêmement variées.

Parmi les techniques les plus usitées, le fractionnement éthanolique permet de séparer de manière sélective les différents constituants du plasma, en faisant précipiter ces derniers par l'action combinée de l'éthanol et d'une faible température (voir Cohn et al. 1946, J. AM. Chem. Soc. 68, 459). L'augmentation des standards thérapeutiques a toutefois nécessité la mise en oeuvre d'étapes de purification additionnelles visant à éliminer les contaminants susceptibles de déclencher des réactions anaphylactiques ou susceptibles d'être délétères ou pathogènes pour l'organisme du patient receveur. Parmi les techniques employées pour effectuer ces étapes de purification complémentaire des produits plasmatiques, les techniques de précipitation et de séparation chromatographiques sont les plus employées.

Il apparaît ainsi que la mise en oeuvre d'une technique de précipitation des protéines en présence d'acide caprylique (aussi dénommé acide octanoïque) permet d'éliminer la plupart des protéines du plasma à l'exception des immunoglobulines (voir Steinbuch et al., Rev. Franç. Et. Clin. Et Biol. 1969, XIV, 1054).

Divers procédés ont également été mis au point pour augmenter la pureté des produits par la mise en oeuvre de techniques chromatographiques. On peut notamment citer les demandes de brevet EP 0 703 922 et WO 99/64462, qui décrivent l'association d'au moins deux étapes successives de chromatographie, l'une par échange d'anions, l'autre par échange de cations. La spécificité de ces procédés est apportée par la propriété des échangeurs d'anions de ne pas retenir les immunoglobulines G, dans les conditions classiques de mise en oeuvre, mais de fixer la plupart des autres protéines co-purifiées au cours des étapes de prépurification.

La demande de brevet WO 02/092632, déposée par la demanderesse, divulgue pour sa part un procédé de préparation de concentrés d'immunoglobulines humaines à partir du plasma, comprenant une étape de fractionnement éthanolique, une étape de précipitation des contaminants lipidiques et protéiques au moyen d'acide caprylique, de phosphate tricalcique ou de bentonite, et une séparation chromatographique sur échangeur d'anions à pH alcalin, au cours de laquelle les immunoglobulines s'absorbent sur le support chromatographique. Le procédé décrit dans demande WO 02/092632 peut également comprendre une étape d'inactivation virale, ainsi qu'une étape de concentration, de filtration et de lyophilisation du concentré d'immunoglobulines obtenu.

La demande WO 2007/077365, déposée par la demanderesse, décrit également un procédé de préparation d'un concentré d'immunoglobulines G appauvri en anticorps anti-A et anti-B comprenant une étape de chromatographie d'affinité effectuée sur un support présentant des oligosaccharides antigéniquement similaires aux groupes sanguins A et B, et comprenant une étape d'élimination virale par filtration. Les concentrés d'IgG obtenus par le procédé décrit dans la demande WO 2007/077365 présentent une teneur en anticorps anti-A et anti-B conforme à un résultat négatif au test de Coombs indirect in vitro et une faible polyréactivité générée par le procédé de fabrication, réduisant de cc fait les risques d'hémolyse et d'effets indésirables résultant de ces anticorps ou de cette polyréactivité.

Le document US5164487 décrit également un procédé de préparation d'un concentré d'immunoglobulines G obtenu à partir du plasma grâce à un procédé comprenant une étape de fractionnement à l'éthanol, une étape de précipitation à l'acide caprylique ; et une étape de chromatographie sur résine échangeuse d'ions.

Le document EP0512883 décrit également un procédé d'obtention d'un concentré de facteur XI par filtration-adsorption sur un filtre de cellulose-perlite chargé négativement, puis une purification par chromatographie sur résine échangeuse d'ions.

La présente invention a pour objet les produits plasmatiques tels que les immunoglobulines spécifiques, les immunoglobulines polyvalentes, les enzymes, les protéines de l'anesthésie et de la réanimation par exemple l'albumine, le fibrinogène ou l'antithrombine.

Les produits plasmatiques purifiés connus restent encore susceptibles de générer des effets indésirables chez les patients, lors de leur administration thérapeutique, en particulier en raison du fait qu'ils ne peuvent a priori être considérés comme totalement dépourvus de facteurs thrombogènes. La présence de ces facteurs thrombogènes FXII, FX, FIX, FVII, FVIII et/ou FXI et de leurs formes activées est susceptible de conduire à la formation de thromboses lorsque le produit plasmatique en question est administré aux patients, et pourrait dans les cas extrêmes aller jusqu'à la mort de ces patients. Les procédés connus dans l'état de la technique ne divulguent pas spécifiquement de méthode pour éliminer de façon satisfaisante ces facteurs thrombogènes, dans la mesure où les propriétés physico-chimiques de ceux-ci et de leurs formes activées, peuvent être extrêmement proches de celles des produits plasmatiques présentant un intérêt thérapeutique, tels que les immunoglobulines par exemple.

Il existe donc un fort besoin pour un procédé de purification permettant de préparer des produits plasmatiques à partir de plasma humain, dans lesquels la quantité finale de facteurs thrombogènes, tels que les facteurs FXI, FXII, FX, FIX, FVII et/ou FVIII, et notamment de FXI, ainsi que de leurs versions activées, est nulle ou si faible qu'elle ne présente aucun risque pour la santé des patients traités avec le produit plasmatique purifié.

### Résumé de l'invention

La demanderesse a ainsi mis au point un procédé permettant avantageusement de préparer un produit plasmatique déplété en un ou plusieurs facteurs thrombogènes.

Par facteurs thrombogènes on entend des facteurs susceptibles de conduire à la formation de thromboses lorsqu'ils sont présents de manière contaminante dans un produit plasmatique. Les facteurs thrombogènes peuvent notamment être des facteurs de coagulation. En particulier les facteurs FVII, le FVIII, le FIX, le FX, FXI et FXII et leurs formes activées sont des facteurs thrombogènes.

Dans un mode de réalisation préféré, le produit plasmatique obtenu par le procédé de l'invention est déplété en un ou plusieurs facteurs thrombogènes. Notamment, le produit plasmatique est déplété en FVII, FVIII, FIX, FX, FXI et/ou FXII et leurs formes activées. En particulier, le produit plasmatique obtenu par le procédé de l'invention est déplété en FVII, FX, FXII et FXI et leurs formes activées. De manière plus particulière, il est déplété en FVII et sa forme activée FVIIa. De manière plus particulière, il est déplété en FX et sa forme activée FXa. De manière plus particulière il est déplété en FXI et sa forme activée FXIa. De manière plus particulière, il est déplété en FXII et sa forme activée FXIIa.

La présente invention décrit que la mise en oeuvre d'une combinaison d'au moins deux étapes choisies parmi un fractionnement éthanolique, une adsorption-filtration sur un filtre de filtration en profondeur, une précipitation à l'acide caprylique et une chromatographie sur résine échangeuse d'ions, permet avantageusement de dépléter le produit plasmatique en un ou plusieurs facteurs thrombogènes et leurs formes activées.

La présente invention décrit également un procédé de préparation d'un produit plasmatique déplété en facteurs thrombogènes, comprenant la combinaison d'au moins deux étapes, de préférence au moins trois étapes, choisies parmi :
- une étape de fractionnement à l'éthanol ;
- une étape de filtration-adsorption ;
- une étape de précipitation à l'acide caprylique ; et
- une étape de chromatographie sur résine échangeuse d'ions.

La présente demande décrit un procédé de préparation d'un produit plasmatique déplété en facteurs thrombogènes comprenant les étapes successives consistant en :
- une étape de fractionnement à l'éthanol ; et
- une étape de filtration-adsorption.

La présente invention décrit un procédé de préparation d'un produit plasmatique déplété en facteurs thrombogènes de comprenant les étapes successives consistant en :
- une étape de fractionnement à l'éthanol ; et
- une étape de précipitation à l'acide caprylique.

La présente invention décrit un procédé de préparation d'un produit plasmatique déplété en facteurs thrombogènes comprenant les étapes successives consistant en :
- une étape de fractionnement à l'éthanol ; et
- une étape de chromatographie sur résine échangeuse d'ions.

La présente invention décrit un procédé de préparation d'un produit plasmatique déplété en facteurs thrombogènes comprenant les étapes successives consistant en :
- une étape de filtration-adsorption ; et
- une étape de fractionnement à l'éthanol

La présente demande décrit un procédé de préparation d'un produit plasmatique déplété en facteurs thrombogènes comprenant les étapes successives consistant en :
- une étape de filtration-adsorption ; et
- une étape de précipitation à l'acide caprylique.

La présente demande décrit un procédé de préparation d'un produit plasmatique déplété en facteurs thrembogène comprenant les étapes successives consistant en :
- une étape de filtration-adsorption ; et
- une étape de cluomatographic sur résine échangeuse d'ions.

La présente demande décrit un procédé de préparation d'un produit plasmatique déplété en facteurs thrombogènes comprenant les étapes successives consistant en :
- une étape de précipitation à l'acide caprylique ; et
- une étape de chromatographie sur résine échangeuse d'ions.

La présente demande décrit un procédé de préparation d'un produit plasmatique déplété en facteurs thrombogènes comprenant les étapes successives consistant en :
- une étape de fractionnement à l'éthanol ;
- une étape de filtration-adsorption ; et
- une étape de précipitation à l'acide caprylique.

La présente invention concerne un procédé de préparation d'un produit plasmatique déplété en facteurs thrombogènes comprenant les étapes successives consistant en :
- une étape de filtration-adsorption ;
- une étape de fractionnement à l'éthanol ; et
- une étape de précipitation à l'acide caprylique.

Dans un mode de réalisation particulier, le procédé de préparation d'un produit plasmatique déplété en facteurs thrombogènes de l'invention comprend les étapes successives consistant en :
- une étape de fractionnement à l'éthanol ;
- une étape de précipitation à l'acide caprylique ; et
- une étape de chromatographie sur résine échangeuse d'ions.

Dans un mode de réalisation particulier, le procédé de préparation d'un produit plasmatique déplété en facteurs thrombogènes de l'invention comprend les étapes successives consistant en :
- une étape de fractionnement à l'éthanol ;
- une étape de filtration-adsorption ; et
- une étape de chromatographie sur résine échangeuse d'ions.

De manière avantageuse, le procédé de préparation de la présente invention comprend en outre une ou plusieurs étapes de chromatographie, de préférence une ou plusieurs chromatographies sur résine échangeuse d'ions.

Dans un mode de réalisation particulier, le procédé de préparation d'un produit plasmatique déplété en facteurs thrombogènes de l'invention comprend les étapes successives consistant en :
- une étape de filtration-adsorption ;
- une étape de précipitation à l'acide caprylique ; et
- une étape de chromatographie sur résine échangeuse d'ions.

Dans un mode de réalisation particulier, le procédé de préparation d'un produit plasmatique déplété en facteurs thrombogènes de l'invention comprend les étapes successives consistant en :
- une étape de fractionnement à l'éthanol ;
- une étape de filtration-adsorption ;
- une étape de précipitation à l'acide caprylique ; et
- une étape de chromatographie sur résine échangeuse d'ions ;
ou consistant en :
- une étape de filtration-adsorption ;
- une étape de fractionnement à l'éthanol ;
- une étape de précipitation à l'acide caprylique ; et
- une étape de chromatographie sur résine échangeuse d'ions.

Dans un mode de réalisation particulier, l'étape de filtration-adsorption est effectuée sur un filtre de filtration en profondeur comprenant de la cellulose et des perlites, ainsi qu'une résine chargée. De manière préférée, le filtre utilisé pour l'étape de filtration-adsorption possède un grade allant de 0,1 à 0,4 µm, de préférence de 0,2 à 0,4 µm, de préférence de 0,25 à 0,35 µm, de préférence de 0,3 µm.

Dans un mode de réalisation particulier, le procédé de préparation de la présente invention comprend une ou deux étapes additionnelles choisies parmi les étapes d'inactivation virale, de préférence par solvant-détergent, et d'élimination virale, de préférence par nanofiltration.

Dans un mode de réalisation particulier, le procédé de préparation de la présente invention comprend une ou deux étapes additionnelles choisies parmi les étapes d'inactivation virale, de préférence par solvant-détergent, et de chromatographie d'affinité sur un support comportant des oligosaccharides antigéniquement similaires aux groupes sanguins A et B.

Dans un mode de réalisation particulier, le procédé de préparation de la présente invention comprend une étape finale de concentration, filtration et d'ajout d'un stabilisant pharmaceutiquement acceptable, puis de conditionnement à l'état de solution stérile et éventuellement de congélation et de lyophilisation.

D'autres caractéristiques et avantages de l'invention apparaîtront à la lecture de la description qui suit d'un mode de réalisation préféré de l'invention, donnée à titre d'exemple.

### Rescription détaillée de modes de réalisation de l'invention

Dans la présente invention, par « produit plasmatique », il est entendu tout composé, toute protéine, telle qu'une immunoglobuline ou l'albumine, le fibrinogène ou l'antithrombine ou tout mélange de protéines, susceptibles d'être purifiés à partir du plasma humain. Par exemple, le produit peut être les immunoglobulines spécifiques, les immunoglobulines polyvalentes, les enzymes, les protéines de l'anesthésie et de la réanimation telles que l'albumine, le fibrinogène ou l'antithrombine. Le produit plasmatique de l'invention correspond de préférence à une immunoglobuline G (ou IgG), une immunoglobuline A (ou IgA), une immunoglobuline M (ou IgM), une immunoglobuline E (ou IgE) ou à un mélange de l'une ou plusieurs de ces dernières, et se présente de préférence sous la forme d'une solution ou d'un concentré. Dans un mode de réalisation particulier, le produit plasmatique de l'invention correspond à un concentré d'immunoglobulines dirigées spécifiquement contre un antigène particulier, comme par exemple des anticorps cytotoxiques anti-rhésus ou anti-D. Dans un autre mode de réalisation, le produit plasmatique de l'invention peut également correspondre à un mélange d'immunoglobulines polyvalentes, composées à 97% d'IgG et dirigées contre une grande diversité d'antigènes. Dans un autre mode de réalisation, le produit plasmatique de l'invention peut en outre correspondre à de l'albumine, du fibrinogène ou de l'antithrombine.

Par « produits obtenus selon le procédé de l'invention » on entend tout produit de type produit plasmatique obtenu de manière directe ou indirecte par le procédé selon l'invention, ou susceptible d'être obtenu par ce procédé.

De manière générale, lorsqu'il est fait référence à un facteur thrombogène il est fait référence aux formes inactivées ou activées de ces facteurs. En effet, les effets thrombogènes de ces facteurs sont particulièrement prononcés lorsque ces derniers se trouvent sous forme activée. Le procédé de la présente invention permet de manière surprenante d'éliminer les facteurs thrombogènes, en particulier lorsqu'ils se trouvent sous leur forme activée. Notamment, lorsqu'il est fait référence au « facteur XI » ou « FXI » dans la présente demande, il est donc entendu le facteur XI sous sa forme inactive et le facteur XI sous sa forme activée (aussi notée FXIa). Il en va de même pour chacun des autres facteurs thrombogènes cités dans la présente demande.

Par « fraction éthanolique » au sens de la présente invention, on entend des fractions plasmatiques obtenues à différents étapes d'un fractionnement éthanolique du plasma tel que connu de l'homme du métier.

Dans le cadre de la présente invention, par « déplété en facteur(s) thrombogène(s)», il est entendu que le produit plasmatique obtenu selon le procédé de l'invention ne contient plus de facteur(s) thrombogène(s), ou contient uniquement des traces non significatives de ce(s) dernier(s). Il en résulte que le produit plasmatique de l'invention ne peut donc pas conduire chez le patient à la formation de thromboses occasionnées par le déclenchement de la coagulation en présence de facteur(s) thrombogène(s) contaminants.

Par « déplété en FVII, en FVIII, en FIX, en FX et/ou en FXII », il est donc entendu que le produit plasmatique obtenu selon le procédé de l'invention ne contient plus de FVII, de FVIII, de FIX, de FX et/ou de FXII, ou qu'il contient uniquement des traces résiduelles de ces derniers. Il en résulte que le produit plasmatique obtenu selon le procédé de l'invention ne peut donc pas conduire à la formation de thromboses occasionnées par le déclenchement de la coagulation en présence de l'un quelconque des Facteurs FVII, FVIII, FIX, FX et/ou FXII.

Dans un mode de réalisation préféré, le produit plasmatique obtenu selon le procédé de l'invention est déplété en FVII et/ou déplété en FX.

Dans un mode de réalisation préféré, le produit plasmatique obtenu selon le procédé de l'invention est déplété en FXI. Par « produit plasmatique déplété en FXI », il est entendu un produit plasmatique, en particulier un produit plasmatique de type immunoglobulines polyvalents présentant moins de 0,8 mU/mg Ig, de préférence moins de 0,5 mU/mg Ig en FXI.

Dans un autre mode de réalisation préféré, le produit plasmatique obtenu selon le procédé de l'invention est déplété en FXII. Par « produit plasmatique déplété en FXII », il est entendu un produit plasmatique, en particulier un produit plasmatique de type immunoglobulines polyvalentes, présentant moins de 20 µU de FXII/mg Ig, de préférence moins de 10 µU de FXII/mg Ig.

Dans un mode de réalisation particulier de la présente invention, le produit plasmatique déplété en un ou plusieurs facteurs thrombogènes peut se présenter sous une forme liquide ou lyophilisée, et/ou être conditionné en présence de stabilisants appropriés. Il peut également être stocké dans l'attente d'une utilisation ultérieure. Les stabilisants ajoutés permettent avantageusement d'assurer la stabilité du produit plasmatique au cours du temps et rendent la lyophilisation possible en évitant la dénaturation du produit plasmatique au cours de cette dernière et au cours de l'entreposage. Les stabilisants utilisés correspondent avantageusement à ceux décrit par la demanderesse dans sa demande de brevet WO 2004/091656, à savoir un mélange d'un sucre alcoolique, de glycine et d'un détergent non-ionique.

Les produits plasmatiques purifiés de l'invention sont destinés à un usage thérapeutique et peuvent faire l'objet d'une administration par voie systémique, orale, parentérale, intraveineuse, intramusculaire, per ou transcutanée, nasale, rectale, perlinguale, oculaire, ou respiratoire. A cet effet, les produits plasmatiques déplétés en un ou plusieurs facteurs thrombogènes de l'invention sont viralement sécurisés, par exemple par un traitement classique d'inactivation virale (par exemple par traitement solvant-détergent) et/ou par un traitement d'élimination virale (par exemple par nanofiltration).

Dans un mode de réalisation préféré, lorsque le produit plasmatique à purifier correspond à des immunoglobulines, la séparation chromatographique est effectuée sur une résine de type échangeur d'anions, de préférence sur une résine de type Diethylaminoethyl (DEAE), Trimethylaminoethyl (TMAE), ou quaternary amine (QAE).

Dans un mode de réalisation particulier de la présente invention, le procédé de l'invention peut comprendre des étapes de purification additionnelles, telles que des chromatographies d'affinité ou des chromatographies hydrophobes.

Lorsque le produit plasmatique purifié de l'invention correspond à des immunoglobulines, le procédé de la présente invention comprend une étape additionnelle de séparation par chromatographie d'affinité sur un support chromatographique comportant des oligosaccharides antigéniquement similaires aux groupes sanguins A et B. Cette étape de purification additionnelle permet avantageusement d'éliminer les anticorps anti-A et anti-B du produit plasmatique purifié obtenu à l'étape précédente. Cette élimination peut être effectuée selon la méthode décrite dans la demande de brevet WO 2007/077365.

Dans un mode de réalisation particulier, le procédé de l'invention comprend également au moins une étape d'inactivation et/ou d'élimination d'agents infectieux, tels que les virus ou les prions. L'inactivation virale est de préférence réalisée par un traitement thermique, ou par un traitement chimique tel qu'un traitement de type solvant-détergent. Le traitement solvant-détergent peut être mis en oeuvre en utilisant par exemple un mélange de tri-n-butylphosphate (TnBP) et d'un détergent choisi parmi le Triton X-100 ou le Tween 80. Il est également possible de procéder à une inactivation virale en soumettant le produit plasmatique purifié à une irradiation aux UV ou aux rayons gamma. L'élimination virale est préférentiellement réalisée par nanofiltration sur des filtres nanométriques ayant une porosité décroissante allant de 100 nm à 15nm. L'étape de nanofiltration permet avantageusement d'éliminer les éventuels pathogènes (virus ou prions, par exemple) qui n'auraient pas été éliminés par le traitement solvant-détergent. L'élimination virale peut être effectuée sur tout filtre approprié, et préférentiellement sur des filtres Planova 35 nm, Planova 20nm et/ou Planova 15 nm. L'étape de nanofiltration peut, le cas échéant, être effectuée après d'éventuelles étapes de concentration et/ou d'ultrafiltration du produit plasmatique purifié.

Dans un mode de réalisation particulier, le procédé de la présente invention peut également comprendre une étape de concentration et/ou d'ultrafiltration du produit plasmatique purifié. Le produit plasmatique purifié peut également être soumis à une étape de filtration stérilisante, et/ou à une étape de congélation et de lyophilisation. Il peut également être stocké dans l'attente d'une utilisation ultérieure. Les stabilisants ajoutés permettent avantageusement d'assurer la stabilité du produit plasmatique au cours du temps et rendent la lyophilisation possible en évitant la dénaturation du produit plasmatique au cours de cette dernière et au cours de l'entreposage. Les stabilisants utilisés correspondent avantageusement à ceux décrit par la demanderesse dans sa demande de brevet WO 2004/091656, à savoir un mélange d'un sucre alcoolique, de glycine et d'un détergent non-ionique.

Dans un mode de réalisation préféré, le procédé de la présente invention comprend la mise en oeuvre successive :
- d'une étape de filtration-adsorption :
- d'une une étape de fractionnement à l'éthanol ;et
- d'une étape de précipitation à l'acide caprylique.

Ces combinaisons d'étapes permettent avantageusement d'éliminer les facteurs thrombogènes présents dans le produit plasmatique de départ, et notamment les facteurs FVII, FVIII, FIX, FX, FXI et/ou FXII.

Ces combinaisons d'étapes, présentant une efficacité comparable, permettent plus particulièrement d'éliminer le facteur FXI, le FVII, le FX et/ou le FXII, jusqu'à atteindre une concentration de facteurs inférieure à leur seuil de détection respectif.

Les exemples suivants illustrent l'invention sans toutefois en limiter la portée.

### Exemples

### Exemple 1 : Elimination du FXI sur filtre

Les différentes étapes de filtration sont réalisées à une température comprise entre 4°C et 10°C. Les filtres chargés sont préalablement rincés avec un minimum de 6mL de solution tampon d'équilibrage (CTS 2.94 g/L, Na₂HPO₄ 1.79 g/L), KH₂PO₄ 0.7 g/L, NaCl 3.5 g/L) par cm² de surface filtrante en appliquant un débit de 7.5mL/h/ cm². Un volume variable de surnageant de cryoprécipitation est filtré. Le débit a été mesuré par pesée de la fraction filtrée à intervalles de temps réguliers. Le débit mesuré après les 5 premières minutes de filtration est considéré comme le débit initial. Selon les essais, le filtre est rincé après injection de la matière première avec un volume variable de solution tampon d'équilibrage permettant d'étudier l'influence du rinçage du filtre sur la rétention du facteur XI et sur la récupération des protéines non adsorbées. Afin de tester l'efficacité de rétention de la filtration-adsorption, le FXI est élué avec la solution tampon d'élution. Des échantillons ont été prélevés sur les fractions homogénéisées départ, filtrat et éluat, puis aliquotés, identifiés et stockés à une température inférieure ou égale à -70°C.

Les résultats obtenus au cours des essais de filtration pour le filtre testé sont présentés dans le tableau ci-dessous et démontrent la capacité d'un filtre de filtration en profondeur comprenant de la cellulose et des perlites, ainsi qu'une résine chargée, par exemple le filtre Sartoclear® P 24 cm², ledit filtre possédant un grade allant de 0,1 à 0,4 µm, à retenir le FXI.

| Filtre testé | Charge (ml/ cm²) | Débit (mL/h/cm²) | Perte FXI dans le filtrat | Rendement de rétention du FXI (Eluat 1M NaCl) | Volume filtrat récupéré (mL/cm²) |
|---|---|---|---|---|---|
| Sartoclear® P 24 cm² | 4.2 | 5 | < seuil détection | 99% | 7.9 |
| Sartoclear® P 24 cm² | 14.6 | 5 | < seuil détection | 72% | 21.5 |

### Exemple 2 : Elimination du FXI par chromatographie

Les différentes étapes de filtration ont été réalisées à une température comprise entre 4°C et 10°C.

Après clarification par passage sur Millipak 0.22µm, le surnageant de cryoprécipitation a été injecté sur une colonne packée avec le gel étudié équilibré avec la solution tampon d'équilibrage (CTS 2.94 g/L, Na₂HPO₄ 1.79 g/L), KH₂PO₄ 0.7 g/L, NaCl 3.5 g/L). Le gel a ensuite été lavé avec la même solution tampon jusqu'à retour à la ligne de base.

Afin de tester l'efficacité de rétention de la chromatographie, le FXI a été ensuite élué par passage de solution tampon d'élution de force ionique très élevée sans étape de prélavage du gel pour éliminer les protéines faiblement fixées sur le gel.

Des échantillons ont été prélevés sur les fractions collectées et homogénéisées, aliquotés, identifiés et stockés à une température inférieure ou égale à -70°C jusqu'aux dosages biochimiques.

Les résultats obtenus au cours des essais de chromatographie, avec différentes colonnes de chromatographie, sont présentés dans le tableau ci-dessous

| Conditions d'essai | Charge FXI appliquée (U/mL de gel) | Débit (mL/h/cm²) | Perte FXI dans le filtrat | Rendement de rétention du FXI |
|---|---|---|---|---|
| SP Sepharose « Big Beads » Colonne K15 packée à 5cm de hauteur de gel 7°C Eluat 1M | 12 | 150 | <21% | 92.5% |
| Streamline SP en lit fixe Colonne K26 (5 cm²) packée à 1cm de hauteur de gel 12°C Eluat 1M | 40 | 60 | 23.6% | 78.3% |
| Streamline SP en lit fluidisé | 63 | 300 | 28.1% | 70.5% |
| Colonne K26 (2 cm²) packée à 5.5cm de hauteur de gel 5.5°C Eluat 1M | | | | |

### Exemple 3

### A : Fractionnement éthanolique, précipitation caprylique et chromatographie

On utilise comme matériau de départ 1 Kg de précipité éthanolique "fraction 1 + fraction II + fraction III", obtenu à partir de plasma selon la méthode de Cohn ou de Kistler et Nitschmann (1962, Vox Sang. 7,414). Ce précipité est remis en suspension en tampon acétate (acétate de sodium-acide acétique) à pH 4,7-4,9, sous agitation, à 20 °C.

On ajoute au précipité remis en suspension de l'acide caprylique. L'addition doit être effectuée lentement, à température ambiante. Le mélange est additionné d'un adjuvant de filtration et le précipité est séparé par filtration au moyen d'un filtre-presse.

Le filtrat est récupéré, clarifié et concentré par ultrafiltration, puis il est soumis à une filtration stérilisante à 0,45 µm et 0,2 µm. Il est ensuite soumis à un traitement d'inactivation virale par solvant/détergent comme décrit par Neurath et Horowitz (brevet US 4,764,369). On utilise un mélange Triton X100/TnBP.

Le mélange est ajusté à 64 g/l de protéines, à pH 6,5. Le contact est maintenu pendant 4 à 6 heures, entre 4 et 25 °C. Ensuite le pH est ajusté à 9 avec du NaOH. Le mélange est ensuite soumis à une chromatographie d'échange d'anions sur colonne. On utilise comme matériau échangeur d'anions du TMAE Fractogel®, chargé dans une colonne de chromatographie, équilibré en tampon glycine-NaCl (glycine 0,676 g/l-NaCl 0,526 g/l) à pH 9. Le mélange est chargé sur la colonne à raison de 50 g de protéines pour 1 litre de gel. Après la charge, la colonne est lavée en tampon glycine-NaCl, pH 9 (le même que pour l'équilibrage). Le lavage est surveillé par la densité optique de l'effluent à 280 nm. Après retour à la ligne de base, la colonne est éluée avec du tampon phosphate à pH 6,2 (hydrogénophosphate disodique-dihydrogénophosphate de sodium). L'éluat, contenant les immunoglobulines G, est ajusté à pH 4,5 et soumis à une ultrafiltration sur cassettes.

La solution est ensuite soumise à une filtration stérilisante à 0,22 µm, puis à une filtration nanométrique sur trois filtres disposés en série et de seuils de rétention décroissants, de 100, 50 et 20 nanomètres. La filtration est suivie d'une ultrafiltration sur cassette pour concentrer la solution finale à 120-150 g/l.

### B : Comparaison d'IgIV par rapport à leur taux en facteur XI et facteur XII

Un concentré d'immunoglobulines préparé selon le procédé décrit en exemple 3A est comparé à d'autres concentrés d'immunoglobulines du marché.

Un concentré d'IgIV préparé selon le procédé décrit en exemple 3A présente un taux de facteur XI inférieur à 0,5 mU/mg Ig alors que les autres concentrés d'immuglobulines du marché présentent des taux de facteur XI supérieurs à 0,8 mU/mg Ig, avec pour certaines un taux de facteur XI supérieur à 2 mU/mg Ig.

Le taux de facteur XII a également été évalué. Les concentrés d'immunoglobulines du marché présentent un taux de facteur XII supérieur à 20 µU/mg Ig, avec pour certaines un taux de facteur XII supérieur à 30 µU/mg Ig, alors qu'un concentré d'IgIV préparé selon le procédé décrit en exemple 1 présente un taux de facteur XII inférieur à 10 µU/mg Ig.

### C : Dosage des facteurs VII et X avant et après le procédé comprenant les étapes de fractionnement éthanolique, précipitation caprylique et chromatographie.

Les teneurs en facteurs VII et X sont mesurées avant et après le procédé tel que décrit en exemple 3A.

Les teneurs dans le plasma (avant le procédé comprenant le fractionnement éthanolique, la précipitation caprylique et la chromatographie) correspondent aux moyennes des valeurs dosées pour six lots de plasma 4500 litres.

| Facteur | | VII | X |
|---|---|---|---|
| **Plasma** | Volume (l) | 4500 | 4500 |
| | CC (EUI/ml) | 0,66 | 0,39 |
| | **Total (EUI)** | **2970000** | **1755000** |
| **P1 - après** | Volume (l) | 431 | 431 |
| | CC (mEUI/ml) | (<)3,1 | (<)0,7 |
| fractionnement éthanolique, précipitation caprylique et chromatographie. | **Total (EUI)** | **1336,1** | **301,7** |
| **Facteur d'élimination (P1/Plasma)** | | **2223** | **5817** |
| **Log** | | (**>**)**3**,**3** | (**>**)**3**,**8** |

| | | | |
|---|---|---|---|
| ***Cc : concentration - (<) : concentration inférieure au seuil de détection ; dans ce cas, le seuil de détection est pris pour les calculs*** | | | |

Après les étapes de fractionnement éthanolique, précipitation caprylique et chromatographie, la teneur en FVII antigène est inférieure à la limite de détection.

Le ratio P1/plasma montre une diminution liée au procédé comprenant les étapes de fractionnement éthanolique, précipitation caprylique et chromatographie supérieure à 3 logs.

Comme pour le facteur VII, après les étapes de fractionnement éthanolique, précipitation caprylique et chromatographie, la teneur en F X antigène est inférieure à la limite de détection.

Le ratio P1/plasma montre une diminution liée au procédé comprenant les étapes de fractionnement éthanolique, précipitation caprylique et chromatographie de près de 4 logs.

L'exemple 3C montre bien qu'un procédé comprenant les étapes de fractionnement éthanolique, précipitation caprylique et chromatographie selon l'invention permet l'obtention d'un produit plasmatique déplété en facteurs thrombogènes.

### Exemple 4 :

### A : Filtration, fractionnement éthanolique et précipitation caprylique.

Du surnageant de cryoprécipitation est filtré sur filtre de filtration en profondeur comprenant de la cellulose et des perlites, ainsi qu'une résine chargée, par exemple le filtre Sartoclear® P 24cm², ledit filtre possédant un grade allant de 0,1 à 0,4 µm.

Le filtrat est soumis à un fractionnement éthanolique selon la méthode de Cohn ou de Kistler et Nitschmann (1962, Vox Sang. 7,414). Le précipité éthanolique "fraction I + fraction II + fraction III" est remis en suspension en tampon acétate (acétate de sodium-acide acétique) à pH 4,7-4,9, sous agitation, à 20 °C.

On ajoute au précipité remis en suspension de l'acide caprylique. L'addition doit être effectuée lentement, à température ambiante. Le mélange est additionné d'un adjuvant de filtration et le précipité est séparé par filtration au moyen d'un filtre-presse.

Le filtrat est récupéré, clarifié et concentré par ultrafiltration, puis il est soumis à une filtration stérilisante à 0,45 µm et 0,2 µm.

### B : Dosage des facteurs VII et X avant et après le procédé comprenant les étapes de filtration, fractionnement éthanolique et précipitation caprylique.

Les teneurs en facteurs VII et X sont mesurées avant et après le procédé tel que décrit en exemple 4A.

Les teneurs avant le procédé correspondent aux moyennes des valeurs dosées pour six lots de plasma 4500 litres.

| Facteur | | VII | X |
|---|---|---|---|
| **Plasma** | Volume (1) | 4500 | 4500 |
| | CC (EUI/ml) | 0,85 | 0,75 |
| | **Total (EUI)** | **3825000** | **3375000** |
| **P1 -** après filtration, fractionnement éthanolique, et précipitation caprylique. | Volume (l) | 436 | 436 |
| | CC (mEUI/ml) | (<)3,1 | (<)0,7 |
| | **Total (EUI)** | **1351,6** | **305,2** |
| **Facteur d'élimination (P1/Plasma)** | | **2830** | **11058** |
| **Log** | | **(>)3,5** | **(>)4,1** |

| | | | |
|---|---|---|---|
| ***Cc : concentration - (<) : concentration inférieure au seuil de détection ; dans ce cas, le seuil de détection est pris pour les calculs*** | | | |

Après les étapes de filtration, fractionnement éthanolique et précipitation caprylique, la teneur en FVII antigène est inférieure à la limite de détection.

Le ratio P1/plasma montre une diminution liée au procédé comprenant les étapes de filtration, fractionnement éthanolique et précipitation caprylique supérieur à 3 logs.

Comme pour le facteur VII, après les étapes de fractionnement éthanolique, précipitation caprylique et chromatographie, la teneur en F X antigène est inférieure à la limite de détection.

Le ratio P1/plasma montre une diminution liée au procédé comprenant les étapes de filtration, fractionnement éthanolique et précipitation caprylique de plus de 4 logs.

L'exemple 4B montre bien qu'un procédé comprenant les étapes de filtration, fractionnement éthanolique et précipitation caprylique selon l'invention permet l'obtention d'un produit plasmatique déplété en facteurs thrombogènes.

## Revendications

1. Procédé de préparation d'un produit plasmatique déplété en un ou plusieurs facteurs thrombogènes, comprenant les étapes successives consistant en :
- une étape de filtration-adsorption ;
- une étape de fractionnement à l'éthanol ; et
- une étape de précipitation à l'acide caprylique.

2. Procédé selon la revendication 1, comprenant une étape additionnelle de chromatographie sur résine échangeuse d'ions.

3. Procédé selon l'une des revendications 1 à 2 dans lequel l'étape de filtration-adsorption est effectuée sur un filtre de filtration en profondeur comprenant de la cellulose et des perlites, ainsi qu'une résine chargée, ledit filtre possédant un grade allant de 0,1 à 0,4 µm, de préférence de 0,2 à 0,4 µm, de préférence de 0,25 à 0,35 µm, de préférence de 0,3 µm.

4. Procédé selon l'une quelconque des revendications 1 à 3, **caractérisé en ce qu'**il comprend une étape additionnelle d'inactivation virale, de préférence par solvant-détergent, et/ou une étape additionnelle d'élimination virale, de préférence par nanofiltration.

5. Procédé selon l'une quelconque des revendications 1 à 4, **caractérisé en ce qu'**il comprend une étape additionnelle de chromatographie d'affinité sur un support comportant des oligosaccharides antigéniquement similaires aux groupes sanguins A et B.

## Patentansprüche

1. Verfahren zur Herstellung eines um einen oder mehreren thrombogenen Faktoren verarmten Plasmamaterials, die aufeinanderfolgenden Schritte umfassend, welche aus:
- einem Schritt der Filtration-Adsorption,
- einem Schritt der Ethanolfraktionierung, und
- einem Schritt der Fällung mittels Caprylsäure
bestehen.

2. Verfahren nach Anspruch 1, einen zusätzlichen Schritt der Chromatographie anhand von Ionenaustauschharz umfassend.

3. Verfahren nach einem der Ansprüche 1-2, in dem der Schritt der Filtration-Adsorption anhand eines Filters für Tiefenfiltration erfolgt, welcher Zellstoff und Perlite sowie einen gefüllten Harz enthält, wobei der Filter ein Grad von 0,1-0,4 µm, vorzugsweise von 0,2-0,4 µm, vorzugsweise von 0,25-0,35 µm, vorzugsweise von 0,3 µm aufweist.

4. Verfahren nach einem der Ansprüche 1-3, **dadurch gekennzeichnet, dass** es einen zusätzlichen Schritt der Virusinaktivierung, vorzugsweise durch Lösemittel-Detergens, und/oder einen zusätzlichen Schritt der Viruselimination, vorzugsweise durch Nanofiltration umfasst.

5. Verfahren nach einem der Ansprüche 1-4, **dadurch gekennzeichnet, dass** es einen zusätzlichen Schritt der Affinitätschromatographie auf einem Träger umfasst, der Oligosaccharide enthält, welche in ihrer Wirkung als Antigen ähnlich den Blutgruppen A und B sind.

## Claims

1. A method for preparing a plasma product depleted of one or more thrombogenic factors, comprising the successive steps consisting of:
- a filtration-adsorption step;
- an ethanol fractionation; and
- a precipitation step with caprylic acid.

2. The method according to claim 1, comprising an additional step of chromatography on ion exchange resin.

3. The method according to claims 1 to 2, wherein the filtration-adsorption step is conducted on a depth filtration filter comprising cellulose and perlites, and a charged resin, the said filter having a grade ranging from 0.1 to 0.4 µm, preferably 0.2 to 0.4 µm, preferably from 0.25 to 0.35 µm, preferably 0.3 µm.

4. The method according to any of claims 1 to 3, **characterized in that** it comprises an additional viral inactivation step, preferably using solvent-detergent, and/or an additional viral removal step preferably by nano filtration.

5. The method according to any of claims 1 to 4 **characterized in that** it comprises an additional affinity chromatography step on a support comprising oligosaccharides having antigenic similarity with the blood groups A and B.
